# EUROPEAN PATENT APPLICATION

(11) **EP 4 524 973 A1**
(43) Date of publication of application: **19.03.2025**
(21) Application number: 23849277.1
(22) Date of filing: 28.07.2023
(51) Int. Cl.: G16B 20/30

(54) **HAPLOTYPE CONSTRUCTION METHOD INDEPENDENT OF PROBAND**

(30) Priority: 05.08.2022 CN 202210935337
(71) Applicant: Suzhou Basecare Medical Device Co., Ltd., Suzhou, Jiangsu 215000 (CN)
(72) Inventor: XU, Ruixia, Suzhou, Jiangsu 215000 (CN); GU, Mengnan, Suzhou, Jiangsu 215000 (CN); SHAN, Wenqi, Suzhou, Jiangsu 215000 (CN); YANG, Yuyan, Suzhou, Jiangsu 215000 (CN); KONG, Lingyin, Suzhou, Jiangsu 215000 (CN); LIANG, Bo, Suzhou, Jiangsu 215000 (CN)
(74) Representative: Buzzi, Notaro & Antonielli d'Oulx S.p.A.
(86) International application number: PCT/CN2023/109731
(87) International publication number: WO 2024/027569

(57) **Abstract**

Disclosed is a haplotype construction method independent of a proband, comprising: performing long fragment sequencing on a DNA sample of one of pathogenic variation carrier parents in a couple; performing whole genome analysis on sequencing data of the pathogenic variation carrier parent to obtain genotype information of the pathogenic variation; screening heterozygous SNPs in the pathogenic variation carrier parent; gathering the heterozygous SNPs for whole genome assembly and typing; according to assembly information of the long fragment where the pathogenic variation is located, obtaining a typing result of the pathogenic variation site; and marking the haplotype of the pathogenic variation carrier parent according to the typing result of the pathogenic site, and constructing a pathogenic/normal haplotype of the pathogenic variation carrier parent. In the present application, only a pathogenic chromosome variation carrier is subjected to long fragment sequencing, and a haplotype of the carrier is constructed. The method can be effectively applied to genetic screening, and accurate haplotyping of the whole chromosome can be realized by means of the SNP interlocking analysis and correction of embryos.

## Description

### TECHNICAL FIELD

The present application belongs to the field of gene detection technology and relates to a haplotype construction method independent of a proband.

### BACKGROUND

Monogenic disorders are a type of disease caused by mutations in a single gene. Such disorders are passed on to later generations following Mendelian laws and are therefore also known as Mendelian diseases. Although conventional prenatal diagnosis during pregnancy can prevent the birth of affected offspring; however, termination the pregnancy brings great pain and psychological burden to pregnant woman and her family. Therefore, it is of great significance and clinical value to fundamentally block the transmission of pathogenic genes of genetic diseases.

Chromosome structural variation includes chromosome translocation and chromosome inversion. The chromosome translocation includes reciprocal translocation and Robertsonian translocation. The incidence rate of balanced translocations in the population is 0.19%. Carriers of balanced reciprocal translocations usually do not have any obvious phenotypic abnormalities, but the balanced reciprocal translocations are closely associated with adverse pregnancy outcomes such as recurrent spontaneous abortion, fetal death, stillbirth, neonatal mental retardation, and other congenital malformations. Currently reported testing techniques for preimplantation genetic testing for monogenic diseases (PGT-M) and preimplantation genetic testing for structural rearrangements (PGT-SR) are similar, including fluorescent in situ hybridization (FISH), array comparative genomic hybridization (arrayCGH), single nucleotide polymorphism array (SNP array), and next-generation sequencing (NGS).

PGT-M and PGT-SR testing rely on the amplification of minute quantity of samples derived from embryos. However, allele drop-out (ADO) may occur during the amplification process, meaning that one of the alleles may be preferentially amplified or fail to amplify entirely, leading to false positive or false negative results. Consequently, clinical practice often utilizes family-based linkage haplotype analysis to enhance the accuracy of preimplantation genetic testing (PGT). Initially, normal/pathogenic haplotypes are constructed based on the samples from a couple and a proband, or reference individuals, which include samples from related relatives who share a blood relationship with the proband. The pathogenic allele from either the father or mother of the embryo or fetus is distinguished based on the proband (or reference individuals), and then the pathogenicity of the embryo or fetus is determined. However, in cases involving unique family structures that no proband or reference individuals are available, the aforementioned method cannot be used to determine whether the embryo or fetus carries pathogenic variants in its genome.

In summary, in clinical practice, some patients with monogenic diseases or carriers of structural variations are unable to provide the genomic samples of a proband or reference for various reasons, or the mutations carried by the couple may be de novo mutations, making it impossible to construct haplotypes through family-based linkage analysis. Furthermore, a small number of patients are unable to construct haplotypes due to a large number of homozygous regions resulting from consanguineous marriages. These factors all limit the application of PGT technology. Therefore, developing haplotype construction methods that do not rely on probands is of great significance in the field of genetic testing technology.

### SUMMARY

The present application provides a haplotype construction method that does not rely on a proband. By sequencing only one chromosome abnormality carrier, the method directly obtains the pathogenic site and its flanking sequence information. This approach addresses the challenges in clinical settings where no proband or unbalanced embryo sample is available for PGT-M/PGT-SR testing, thereby broadening the applicability of PGT-M/PGT-SR.

In a first aspect, the present application provides a method for constructing a haplotype that does not rely on a proband, and the method includes the following steps:
performing long fragment sequencing on a DNA sample from one of pathogenic variation carrier parents in a couple;
performing whole-genome analysis on sequencing data of the pathogenic variation carrier parent to obtain genotype information of the pathogenic variation;
screening heterozygous single nucleotide polymorphisms (SNPs) in the pathogenic variation carrier parent; and
gathering the SNPs for whole-genome assembly and phasing; obtaining a phasing result of a pathogenic variation site according to assembly information of a long fragment where the pathogenic variation is located; and marking a haplotype of the pathogenic variation carrier parent according to the phasing result of the pathogenic sites, and constructing a pathogenic/normal haplotype for the pathogenic variation carrier parent.

In the present application, a haplotype construction method that does not rely on a proband is designed. By only performing long fragment sequencing on a chromosome pathogenic variation carrier parent and performing site screening based on SNPs, a pathogenic/normal haplotype of a chromosomal abnormality carrier can be obtained without relying on a proband or a reference, and thus the method can be applied to determine whether an embryo inherits the pathogenic variation, thereby assisting in screening a normal embryo for implantation and avoiding the birth of a progeny carrying the pathogenic variation.

In the present application, the long fragment sequencing may be single-molecule long-read sequencing or may be long-read sequencing indirectly implemented by introducing a sequence tag to a large fragment.

Preferably, the genotype information of the pathogenic variation includes structural variation, point mutation or Indel, and duplication.

Preferably, the method of marking the haplotype of the parent includes marking the haplotype of the parent with a specific color.

In the present application, the haplotype of the parent may be marked with a color as required. For example, the pathogenic variation-carrying chromosome strand is marked with blue in a specific embodiment of the present application.

Preferably, the method further includes a step of extracting the genomic DNA of the couple.

As a preferred technical solution, the haplotype construction method that does not rely on a proband includes the following steps:
(1) performing long fragment sequencing on a DNA sample from one of the pathogenic variation carrier parents in a couple;
(2) performing whole-genome analysis on sequencing data of the pathogenic variation carrier parent to obtain genotype information of the pathogenic variation;
(3) screening heterozygous SNPs in the pathogenic variation carrier parent;
(4) gathering the SNPs for whole-genome assembly and phasing; obtaining a phasing result of a pathogenic variation site according to assembly information of a long fragment where the pathogenic variation is located; and marking a haplotype of the pathogenic variation carrier parent according to the phasing result of the pathogenic sites, and constructing a pathogenic/normal haplotype for the pathogenic variation carrier parent.

In a second aspect, the present application provides an apparatus for screening an embryo for genetic diseases and chromosomal abnormalities. The apparatus includes a haplotype constructing unit and a screening unit. The haplotype constructing unit is used for performing the haplotype construction method independent of a proband described in the first aspect. The screening unit is used for performing whole-genome SNP analysis on a whole-genome amplification product of a progeny embryo sample and performing SNP linkage analysis on a homozygous genotype of the progeny embryo according to a haplotype result of a pathogenic variation carrier parent to obtain a phasing result of a single chromosome strand of the progeny embryo.

In the present application, a pathogenic/normal haplotype of a chromosomal abnormality carrier is constructed without relying on a proband or a reference based on the haplotype construction method independent of a proband, accurate haplotyping of the whole chromosome can be achieved by performing SNP linkage analysis and correction on embryos, and then whether the embryo carries a pathogenic mutation is determined, thereby helping to screen chromosomally normal embryos for patients who cannot receive conventional PGT-M/PGT-SR treatment in the case where a haplotype cannot be constructed due to various factors such as the lack of a proband sample, the presence of a de novo mutation or the existence of a large number of homologous regions due to the consanguineous marriage.

In the present application, the whole-genome amplification (WGA) may be PCR-based whole-genome amplification, whole-genome amplification based on isothermal amplification, or whole-genome amplification based on multiple annealing and looping-based amplification cycles.

Preferably, the screening unit is further used for performing a step of performing whole-genome amplification on the embryo sample, and the embryo sample includes an embryo biopsy sample.

Preferably, the embryo biopsy sample includes a blastomere biopsy sample or a blastocyst trophectoderm biopsy sample.

Preferably, the screening unit is further used for performing a step of correcting an erroneous assembly.

In the present application, since the haplotype results of embryos will have the same breakpoints due to an erroneously assembled block in the parent, the above erroneous assembly is corrected to obtain an accurate phasing result.

As a preferred technical solution, the haplotype constructing unit is used for performing the following steps:
(1) performing long fragment sequencing on a DNA sample from one of the pathogenic variation carrier parents in a couple;
(2) performing whole-genome analysis on sequencing data of the pathogenic variation carrier parent to obtain genotype information of the pathogenic variation;
(3) screening heterozygous SNPs in the pathogenic variation carrier parent;
(4) gathering the SNPs for whole-genome assembly and phasing; obtaining a phasing result of a pathogenic variation site according to assembly information of a long fragment where the pathogenic variation is located; and marking a haplotype of the pathogenic variation carrier parent according to the phasing result of the pathogenic sites, and constructing a pathogenic/normal haplotype for the pathogenic variation carrier parent.

The screening unit is used for performing the following steps:
(1) performing whole-genome SNP analysis on a whole-genome amplification product of a progeny embryo sample;
(2) performing SNP linkage analysis on a homozygous genotype of the progeny embryo according to a haplotype result of the pathogenic variation carrier parent and correcting an erroneous assembly to obtain a phasing result of a single chromosome strand of the progeny embryo.

Compared with the prior techniques, the present application has the following beneficial effects.
(1) No need for probands and family-based linkage haplotypes: Through the haplotype construction method independent of a proband of the present application, only a pathogenic chromosome variation carrier is subjected to long fragment sequencing to construct a haplotype of the carrier, and thus, the method can be applied to genetic screening; accurate haplotyping of a whole chromosome can be achieved by performing SNP linkage analysis and correction on embryos, and then whether the embryo carries a pathogenic mutation is determined.
(2) Strong versatility: Various kinds of variation information within the whole genome of the sample can be obtained by performing sequencing on the sample only once, and when the obtained information is applied to different analysis procedures, to obtain results of PGT-A, PGT-M, and PGT-SR and other embryos abnormality status so there is no need to carry out individual design for different chromosome abnormalities.
(3) Comprehensive detection: Through high-precision long fragment sequencing, high-accuracy SNP/InDel/CNV/SV variation detection results and haplotype phasing results can be obtained by performing sequencing once. The detection is more accurate, has a wider range, and is suitable for analysis of sources of variation such as translocations, inversions, chromosome aneuploidy, deletions, duplications, single-gene disorders, CNV, etc.
(4) High sensitivity and precision: Through high-precision long fragment sequencing, balanced translocation breakpoints throughout the whole genome, including those in complex regions of the genome, can be easily obtained, and the breakpoints can be located to single bases, thereby significantly improving the sensitivity and precision of structural variation (SV) detection.
(5) There is no need to analyze the CNV and structural variation of the progeny, thereby simplifying the analysis steps and reducing manual interpretation.

### BRIEF DESCRIPTION OF DRAWINGS

FIG. 1 shows the detection results of an analysis method applied to a haplotype construction method independent of a proband of the present application in Example 1.
FIG. 2 shows the detection results of the routine PGH family-based linkage analysis method in Comparative Example 1.
FIG. 3 shows the detection results of an analysis method applied to a haplotype construction method independent of a proband of the present application in Example 2.
FIG. 4 shows the detection results of the routine PGH family-based linkage analysis method in Comparative Example 2.

### DETAILED DESCRIPTION

To further elaborate on the technical means adopted and effects achieved in the present application, the present application will be further described below in conjunction with examples and drawings. It is to be understood that the specific examples set forth below are intended to explain the present application and are not intended to limit the present application.

Experiments without specific techniques or conditions specified in the examples are conducted according to techniques or conditions described in the literature in the techniques or according to the specifications of products. The reagents or instruments used herein without manufacturers specified are conventional products commercially available from proper channels.

### Example 1

This example provides a method for haplotype construction of balanced translocations without depending on a proband or reference individuals, and SNP linkage analysis of the embryos was performed for the preimplantation genetic testing for structural rearrangements.

A family with chromosomal balanced translocations who have undergone assisted reproductive procedures (with chromosomal karyotype results available). The family chromosomal karyotypes of the patient are shown in Table 1. Peripheral blood samples (5 mL each) from the balanced translocation carrier, their spouse, and the patient's immediate family members should be collected in EDTA-anticoagulated vials. The whole-genome amplification products of embryo biopsy samples of the couple were obtained, respectively. Genomic DNAs were extracted from the peripheral blood samples of the family according to the high-molecular-weight DNA extraction method in the art.

**Table 1**

| Sample information | Relationship | Karyotype |
|---|---|---|
| CJJ (Male) | Father | 46,XY |
| CYJ | Mother | 46,XX,t(3;19)(q22;q13.2) |
| (Female) | | |
| CR (Mother of the female) | Mother of the female | 46,XX,t(3;19)(q23;q13.3) |
| CYJ01E (Embryo) | Embryo 1 | dup(3)(q22.3q29)(60.76Mb); dup(19)(p13.3q13.32)(45.34Mb); unbalanced translocation, non-transplantable |
| CYJ02E (Embryo) | Embryo 2 | t(3:19)(g22.3:q13.32); balanced translocation, transplantable |

1. Library construction and long fragments sequencing on a single sample from the balanced translocation carrier
   Library construction and sequencing were performed on genomic DNA samples from the balanced translocation carrier CYJ, following the instructions of a long-fragment sequencing platform.
2. Embryo SNP genotype testing using a PGH array
   The SNP genotype testing was performed using a PGH array. Each PGH array contains 690,000 SNPs, comprehensively covering 23 pairs of human chromosomes. The SNP genotype testing was conducted on the WGA products derived from trophectoderm biopsy cells of two progeny embryos from the patient using a PGH array. The specific experimental methods were conducted according to the instruction manual, and the details are not repeated here.
3. Haplotypes construction for the a single sample from balanced translocation carrier
   (1) Genotyping of the balanced translocation carrier: Whole-genome analysis was performed on the raw data from long-fragment sequencing of the balanced translocation carrier to obtain genotype information for pathogenic variations (including structural variation, point mutations, indels and duplications).
   (2) Identifying for informative SNP sites: heterozygous SNPs were screened in the balanced chromosome translocation carrier.
   (3) Constructing the haplotype of the balanced translocation carrier: The heterozygous SNPs identified in step (2) were gathered for whole-genome assembly and phasing; a translocation/normal haplotype of the balanced translocation carrier parent was obtained; and the haplotype of the parent was marked with colors according to the phasing result of the balanced translocation (blue for the chromosome chain carrying the balanced translocation).
4. Haplotype construction of progeny embryos o: SNP linkage analysis was performed on a homozygous genotype of the embryo according to a haplotype result of the balanced translocation carrier parent to obtain a phasing result of a single chromosome strand of the embryo; since the haplotype results of embryos had the same breakpoints due to an erroneously assembled block in the parent, the above erroneous assembly was corrected to obtain an accurate phasing result.

### Example 2

This example provides a method for constructing a haplotype of a single-gene disorder independently of a proband or a reference, and SNP linkage analysis of an embryo was performed for the preimplantation genetic testing for monogenic diseases.

For a family having a single-gene disorder patient (which was proved by existing gene testing results), who received assisted reproduction, gene testing results of the patient from the family are shown in Table 2. 5 mL of peripheral blood was extracted from the single-gene disorder patient, the spouse of the patient, and an immediate family member of the patient, respectively, and then stored in EDTA anticoagulation blood collection tubes. The whole-genome amplification products of embryo biopsy samples from the couple were obtained, respectively. Genomic DNAs were extracted from the peripheral blood samples of the family according to the high-molecular-weight DNA extraction method in the art.

**Table 2**

| Family Sample ID. | Relationship | Genetic testing result |
|---|---|---|
| Thalassemia family-FO | Father | --SEA/αα, βN/βN |
| Thalassemia family-MO | Mother | αα/αα, βN/βN |
| Thalassemia family-R1 | Reference | --SEA/αα, βN/βN |
| Thalassemia family-embryo01 | Progeny | --SEA/αα, βN/βN |
| Thalassemia family-embryo02 | Progeny | --SEA/αα, βN/βN |

1. Library construction and long-fragment sequencing on a single sample from the single-gene disorder patient.
   Library construction and sequencing were performed on the genomic DNA samples in accordance with the instructions of a long-fragment sequencing platform.
2. Embryo SNP genotype testing using a PGH array
   The SNP genotype testing was performed on the WGA products of trophectoderm biopsy cells from two progeny embryos of the patient using a PGH array.
3. Single sample haplotype construction for the single-gene disorder carrier
   (1) Genotyping of the single-gene disorder carrier: The whole-genome analysis was performed on the raw data of long-fragment sequencing for the single-gene disorder carrier to obtain the genotype information of the pathogenic variation (including point mutations, and insertions, deletions and duplications).
   (2) Identifying of SNP sites: heterozygous SNPs were screened in the single-gene disorder carrier.
   (3) Constructing the haplotype of the single-gene disorder carrier: The heterozygous SNPs identified in step (2) were gathered for whole-genome assembly and phasing; a pathogenic/normal haplotype of the single-gene disorder carrier parent was obtained; and the haplotype of the parent was marked with a color according to the phasing result of the single-gene disorder pathogenic variation (the chromosome strand carrying the single-gene disorder pathogenic variation was blue).
4. Haplotype construction of progeny embryo: SNP linkage analysis was performed on a homozygous genotype of the embryo according to a haplotype result of the single-gene disorder carrier parent to obtain a phasing result of a single chromosome strand of the embryo; since the haplotype results of embryos had the same breakpoints due to an erroneously assembled block in the parent, the above erroneous assembly was corrected to obtain an accurate phasing result.

### Comparative Example 1

In this comparative example, the routine PGH testing was performed on the samples of the balanced translocation family.

### 1. Family-based haplotype construction

(1) Sample genotyping: SNP genotype testing was performed on samples of the male (normal), female (balanced translocation carrier), and the mother of the female (reference) and WGA products of two progeny embryo biopsy samples of the couple. For the specific experimental methods, reference was made to the instructions, and the details are not repeated here.
(2) Identifying of informative SNPs sites: SNP sites that were heterozygous in the balanced chromosome translocation carrier and homozygous in both the spouse of the carrier and the reference were selected. Informative SNPs covering chromosome translocation breakpoints, informative SNPs on the translocation chromosome, and informative SNPs on a normal homologous chromosome were selected. At least one informative SNP per Mb of the chromosome were selected. In the region covering the breakpoints, the informative SNPs were selected within the range of 2 Mb upstream and downstream of the breakpoints.
(3) Construction of family-based linkage haplotypes: The informative SNP sites identified in step (2) were gathered, and the family-based linkage analysis was performed to obtain a haplotype of the covering two breakpoints region, a haplotype of the whole translocation chromosome, and a haplotype of the whole homologous chromosome. The set of different chromosome haplotypes is called family-based haplotypes.

2. Distinguishing between an embryo carrying balanced translocation or an embryo carrying a normal chromosome.

The haplotype information of the breakpoints region in the embryo (blastocyst) cells was compared with the chromosome haplotype information of the reference sample to distinguish whether the embryo carried balanced translocation.

### Comparative Example 2

In this comparative example, the routine PGH testing was performed on the family of the single-gene disorder patient.

### 1. Family-based haplotype construction

(1) Sample genotyping: Large-scale SNP genotype testing was performed on samples of the couple with the single-gene disorder and samples of proband.
(2) Identifying of informative SNP sites: SNP sites that were heterozygous in the single-gene disorder carrier and homozygous in both the spouse of the carrier and the proband were selected. The informative SNPs covering the range of 2 Mb upstream and downstream of the pathogenic mutation were selected.
(3) Construction the haplotype of reference sample in family-based linkage haplotyping: The informative SNP sites identified in step (2) were gathered, and the family-based linkage analysis was performed to obtain a haplotype of the region covering the pathogenic mutation, and the haplotype of reference sample in family-based linkage haplotypes were obtained.

### 2. Distinguishing between an embryo carrying the signal-gene disorder pathogenic variation and a normal embryo

The haplotype information of the pathogenic variation region in embryonic (blastocyst) cells was compared with the haplotype information of the reference sample to distinguish whether the embryo carried the pathogenic variation.

### Result Comparison

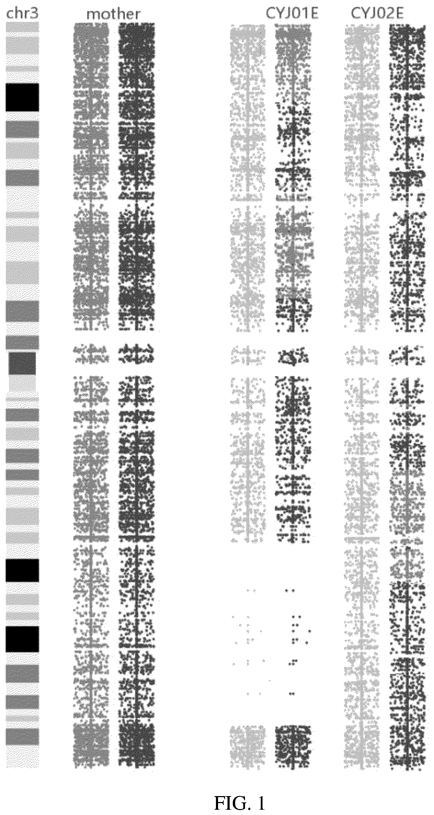

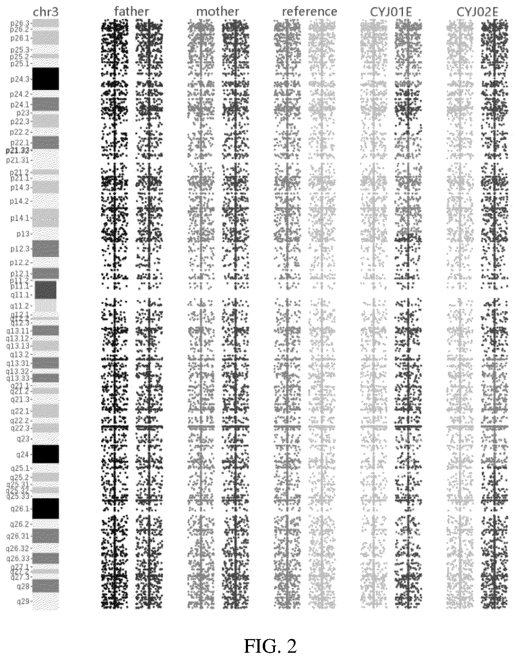

The phasing results of the samples of the balanced translocation family detected using the method of the present application in Example 1 are shown in FIG. 1. The blank region on the long arm of the CYJ01E chromosome is the region of unbalanced translocation, and the starting and ending sites of the region are the breakpoints; the yellow block on the long arm of the CYJ02E chromosome crosses over the breakpoints, and thus the CYJ02E is a balanced translocation carrier. The results of the routine PGH family-based linkage analysis method in Comparative Example 1 are shown in FIG. 2. The results of the method of the present application in Example 1 are compared with the results of the routine PGH family-based linkage analysis method in Comparative Example 1, and the comparison results are shown in Table 3. The karyotype results of the female and the mother of the female in the balanced translocation family are balanced translocation carriers. The detection results of the method of the present application are consistent with the results of the karyotypes and PGH results of the embryos, and the results of breakpoints of the carrier are consistent with the PGH results of the embryos, indicating that the present application can construct haplotypes independently of probands or references and can be effectively applied to genetic screening.

**Table 3**

| Sample information | Karyotype information | BMP detection results | Breakpoints in the method of the present application (SVBND results) | Routine PGH family-based linkage analysis results |
|---|---|---|---|---|
| CJJ (Male) | NA | NA | NA | arr(1-22)x2.(X,N)x1 |
| CYJ (Female) | 46,XX,t(3;19) (q22;q13.2) | 46,XX,t(3;1 9)(q22;q13. 32) | 3:137490474-19:459937 02; | arr(1-22)x2.(X,N)x1 |
| | | | 3:137490507-19:459936 803 | |
| CR (Mother of the female) | 46,XX,t(3;19) (q22.3:q13.2) | 46,XX,t(3;1 9)(q22.3:q1 3.32) | 3:137490500-19:459937 01; | arr(1-22)x2.(X,N)x1 |
| | | | 3:137490507-19:459936 79 | |
| CYJ01E (Embryo) | | t(3;19)(q22. 3;q13.32) | 3:137490507-19:459936 803 | dup(3)(q22.3q29)(60. 76Mb); dup(19)(p13.3q13.32)( 45.34Mb); unbalanced translocation, non-transplantable |
| CYJ02E (Embryo) | | t(3; 19)(q22. 3;q13.32) | 3:137490507-19:459936 79 | arr(1-22)x2.(XN)x1 balanced translocation, transplantable |

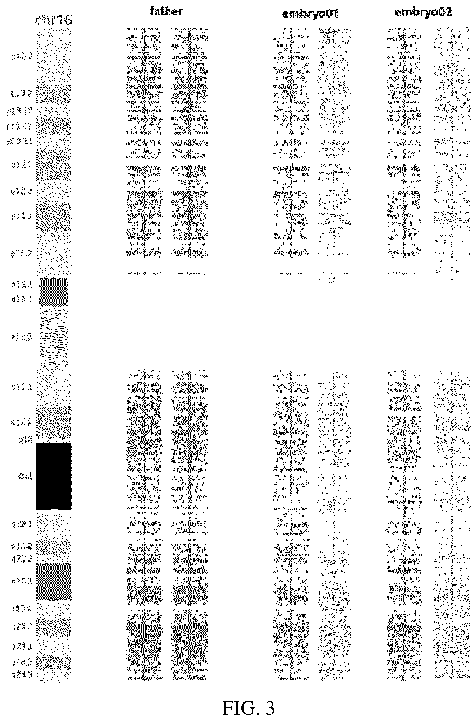

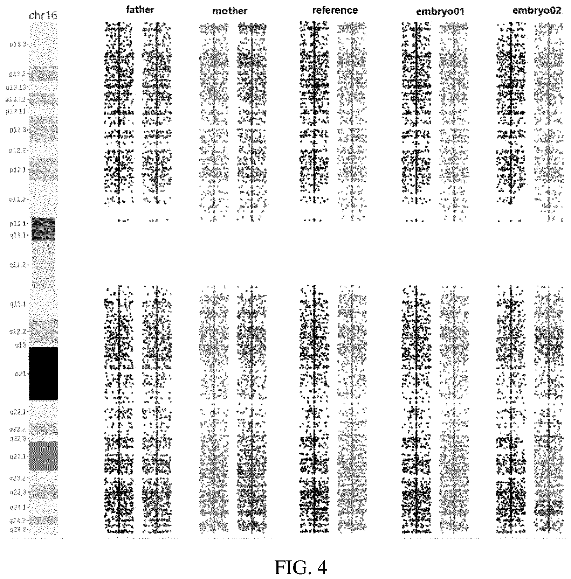

The phasing results of the single-gene disorder family samples detected by the method of the present application in Example 2 are shown in FIG. 3. The results of the routine PGH family-based linkage analysis method in Comparative Example 2 are shown in FIG. 4. The results of the method of the present application in Example 1 are compared with the results of the routine PGH family-based linkage analysis method in Comparative Example 1 (Table 4). The single sample haplotype phasing results of the method of the present application are consistent with the PGH phasing results, and the progenies all carry alpha-thalassemia mutation.

**Table 4**

| Family sample ID | Relationship | Routine PGH family-based linkage analysis results | Detection results of the method of the present application |
|---|---|---|---|
| Thalassemia family-FO | Father | --^{SEA}/αα | --^{SEA}/αα |
| Thalassemia family-MO | Mother | αα/αα, βN/βN | αα/αα, βN/βN |
| Thalassemia family-R1 | Reference | --SEA/αα, βN/βN | --SEA/αα, βN/βN |
| Thalassemia family progeny-S1 | Embryo | --SEA/αα, βN/βN | --SEA/αα, βN/βN |
| Thalassemia family progeny-S2 | Embryo | --SEA/αα, βN/βN | --SEA/αα, βN/βN |

In summary, the present application provides a haplotype construction method that does not depend on a proband or a reference. Only a pathogenic chromosome variation carrier is subjected to long fragment sequencing to construct a haplotype of the carrier. Therefore, the method can be effectively applied to genetic screening, accurate haplotyping of the whole chromosome can be achieved by performing SNP linkage analysis and correction on embryos, and then whether the embryo carries a pathogenic mutation can be determined, thereby helping to screen normal embryos for patients who cannot receive conventional PGT-M/PGT-SR treatment in the case where a haplotype cannot be constructed due to various factors such as the lack of a proband sample, the presence of a de novo mutation or the existence of a large number of homologous regions due to the consanguineous marriage.

The applicant has stated that although the detailed method of the present application is described through the examples described above, the present application is not limited to the detailed method described above, which means that the implementation of the present application does not necessarily depend on the detailed method described above. It is to be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

## Claims

1. A haplotype construction method independent of a proband, comprising the following steps:
(1) performing long fragment sequencing on a DNA sample from one of pathogenic variation carrier parents in a couple;
(2) performing whole-genome analysis on sequencing data of the pathogenic variation carrier parent to obtain genotype information of the pathogenic variation;
(3) screening heterozygous SNPs in the pathogenic variation carrier parent; and
(4) gathering the SNPs for whole-genome assembly and phasing; obtaining a phasing result of a pathogenic variation site according to assembly information of a long fragment where the pathogenic variation is located; and marking a haplotype of the pathogenic variation carrier parent according to the phasing result of the pathogenic sites, and constructing a pathogenic/normal haplotype for the pathogenic variation carrier parent;
wherein in step (1), only the pathogenic variation carrier parent is subjected to the long fragment sequencing, and no detection is performed on a normal parent in the couple, a progeny carrying a pathogenic gene, a relative of the pathogenic variation carrier parent or an embryo with a chromosomal abnormality.

2. An apparatus for screening an embryo for genetic diseases and chromosomal abnormalities, comprising a haplotype constructing unit and a screening unit;
wherein the haplotype constructing unit is used for performing the haplotype construction method independent of a proband according to claim 1; and
the screening unit is used for:
performing whole-genome SNP analysis on a whole-genome amplification product of a progenyembryo sample; and
performing SNP linkage analysis on a homozygous genotype of the progeny embryo according to a haplotype result of a pathogenic variation carrier parent to obtain a phasing result of a single chromosome strand of the progeny embryo.

3. The apparatus for screening an embryo for genetic diseases and chromosomal abnormalities according to claim 2, wherein the screening unit is further used for performing a step of performing whole-genome amplification on the embryo sample, and the embryo sample comprises an embryo biopsy sample;
wherein the embryo biopsy sample comprises a blastomere biopsy sample or a blastocyst trophectoderm biopsy sample.

4. The apparatus for screening an embryo for genetic diseases and chromosomal abnormalities according to claim 2, wherein the screening unit is further used for performing a step of correcting an erroneous assembly.

5. The apparatus for screening an embryo for genetic diseases and chromosomal abnormalities according to any one of claims 2 to 4, wherein the haplotype constructing unit is used for performing the following steps:
(1) performing long fragment sequencing on a DNA sample from one of pathogenic variation carrier parents in a couple;
(2) performing whole-genome analysis on sequencing data of the pathogenic variation carrier parent to obtain genotype information of the pathogenic variation;
(3) screening heterozygous SNPs in the pathogenic variation carrier parent; and
(4) gathering the SNPs for whole-genome assembly and phasing; obtaining a phasing result of a pathogenic variation site according to assembly information of a long fragment where the pathogenic variation is located; and marking a haplotype of the pathogenic variation carrier parent according to the phasing result of the pathogenic sites, and constructing a pathogenic/normal haplotype for the pathogenic variation carrier parent;
wherein in step (1), only the pathogenic variation carrier parent is subjected to the long fragment sequencing, and no detection is performed on a normal parent in the couple, a progeny carrying a pathogenic gene, a relative of the pathogenic variation carrier parent or an embryo with a chromosomal abnormality; and
the screening unit is used for performing the following steps:
(1) performing whole-genome SNP analysis on a whole-genome amplification product of a progeny embryo sample; and
(2) performing SNP linkage analysis on a homozygous genotype of the progeny embryo according to a haplotype result of the pathogenic variation carrier parent and correcting an erroneous assembly to obtain a phasing result of a single chromosome strand of the progeny embryo.
